# EUROPEAN PATENT APPLICATION

(11) **EP 4 623 848 A1**
(43) Date of publication of application: **01.10.2025**
(21) Application number: 25162280.9
(22) Date of filing: 07.03.2025
(51) Int. Cl.: A61B 34/00, A61B 90/00, A61B 34/30, A61B 17/00, A61B 34/20

(54) **SURGICAL ROBOT, ROBOTIC SURGICAL SYSTEM, CONTROL METHOD FOR SURGICAL ROBOT, PROGRAM, AND STORAGE MEDIUM**

(30) Priority: 26.03.2024 JP 2024049777
(71) Applicant: KAWASAKI JUKOGYO KABUSHIKI KAISHA, Kobe-shi, Hyogo 650-8670 (JP)
(72) Inventor: MIZOHATA, Yuichi, Kobe-shi, Hyogo, 650-8670 (JP); ICHII, Tetsuo, Kobe-shi, Hyogo, 650-8670 (JP); YAMAMORI, Hirofumi, Kobe-shi, Hyogo, 650-8670 (JP)
(74) Representative: Hoffmann Eitle

(57) **Abstract**

A surgical robot (100) includes a controller (310) configured or programmed to move a holder (55) to a first replacement position when a detector (53a) detects that a first instrument (2) has been removed, and the controller (310) is configured or programmed to perform a control to move the holder from the first replacement position to a second replacement position based on an input to an operation tool (62).

## Description

### BACKGROUND OF THE INVENTION

### Field of the Invention

The present disclosure relates to a surgical robot, a robotic surgical system, a control method for a surgical robot, a program, and a storage medium, and more particularly, it relates to a surgical robot including, at the distal end thereof, a holder to which an instrument is attached, a robotic surgical system, a control method for a surgical robot, a program, and a storage medium. Description of the Background Art

Conventionally, a surgical robot including, at the distal end thereof, a holder to which an instrument is attached is known.

U.S. Patent No. 6,645,196 discloses a robot system (surgical robot) including an operator-side apparatus, a patient-side apparatus including a plurality of robot arms, a controller, and surgical instruments. The surgical instruments are attached to the robot arms, and surgery is performed. U.S. Patent No. 6,645,196 also discloses a method for replacing the surgical instruments during surgery. Each of the robot arms includes a linear motion mechanism. The controller controls the linear motion mechanism such that the instrument after replacement moves to the same position as the instrument before replacement.

However, when the surgical instruments are replaced during surgery, the robot arms may take various postures depending on the surgical procedure and the situation of the surgery. Depending on the postures of the robot arms at the time of replacement, it may be difficult for a surgeon to replace the surgical instruments, and the replacement work may take time. Therefore, a surgical robot that allows instruments to be replaced easily and quickly is desired.

### SUMMARY OF THE INVENTION

The present disclosure is intended to solve the above problems. The present disclosure aims to provide a surgical robot, a robotic surgical system, a control method for a surgical robot, a program, and a storage medium that each allow an instrument to be replaced easily and quickly no matter what posture a robot arm is in.

A surgical robot according to a first aspect of the present disclosure includes a robot arm including a holder to allow a first instrument to be attached thereto and detached therefrom, an arm operation unit on the robot arm including an operation tool to operate the robot arm, a detector in the holder to detect attachment and detachment of the first instrument to and from the holder, and a controller configured or programmed to move the holder to a first replacement position when the detector detects that the first instrument has been removed. The controller is configured or programmed to perform a control to move the holder from the first replacement position to a second replacement position based on an input to the operation tool.

In the surgical robot according to the first aspect of the present disclosure, the controller is configured or programmed to perform a control to move the holder to the first replacement position when the detector detects that the first instrument has been removed, and move the holder from the first replacement position to the second replacement position based on the input to the operation tool. Accordingly, when a surgical staff removes the first instrument in order to replace the first instrument during surgery, the robot arm can be operated using the operation tool arranged on the robot arm. Thus, the surgical staff can move the robot arm to a posture in which the first instrument can be easily replaced. Consequently, the surgical staff can easily and quickly replace the first instrument.

A robotic surgical system according to a second aspect of the present disclosure includes a surgical robot, and an operation apparatus to operate the surgical robot. The surgical robot includes a robot arm including a holder to allow a first instrument to be attached thereto and detached therefrom, an arm operation unit on the robot arm including an operation tool to operate the robot arm, a detector in the holder to detect attachment and detachment of the first instrument to and from the holder, and a controller configured or programmed to move the holder to a first replacement position when the detector detects that the first instrument has been removed. The controller is configured or programmed to perform a control to move the holder from the first replacement position to a second replacement position based on an input to the operation tool.

In the robotic surgical system according to the second aspect of the present disclosure, the controller is configured or programmed to perform a control to move the holder to the first replacement position when the detector detects that the first instrument has been removed, and move the holder from the first replacement position to the second replacement position based on the input to the operation tool. Accordingly, when a surgical staff removes the first instrument in order to replace the first instrument during surgery, the robot arm can be operated using the operation tool arranged on the robot arm. Thus, the surgical staff can move the robot arm to a posture in which the first instrument can be easily replaced. Consequently, the surgical staff can easily and quickly replace the first instrument.

A control method for a surgical robot according to a third aspect of the present disclosure is a control method for a surgical robot in which an instrument is replaced during surgery, and includes performing a control to detect that the instrument has been removed during the surgery, performing a control to move, to a first replacement position, a holder configured to allow the instrument to be attached and detached thereto and therefrom when it is detected that the instrument has been removed, and performing a control to move the holder from the first replacement position to a second replacement position based on an input to an operation tool configured to operate a robot arm of the surgical robot.

In the control method for the surgical robot according to the third aspect of the present disclosure includes performing a control to move, to the first replacement position, the holder configured to allow the instrument to be attached and detached thereto and therefrom when it is detected that the instrument has been removed, and performing a control to move the holder from the first replacement position to the second replacement position based on the input to the operation tool configured to operate the robot arm of the surgical robot. Accordingly, when a surgical staff removes the instrument of the robot arm in order to replace the instrument during the surgery, the robot arm can be operated using the operation tool arranged on the robot arm. Thus, the surgical staff can move the robot arm to a posture in which the instrument can be easily replaced. Consequently, it is possible to provide the control method for a surgical robot that allows the surgical staff to replace the instrument easily and quickly.

According to the present disclosure, it is possible to replace the instrument easily and quickly no matter what posture the robot arm is in.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

FIG. 1 is a diagram showing the configuration of a robotic surgical system according to an embodiment.
FIG. 2 is a diagram showing a display of a medical cart according to the embodiment.
FIG. 3 is a diagram showing the configuration of the medical cart according to the embodiment.
FIG. 4 is a diagram showing the configuration of a robot arm according to the embodiment.
FIG. 5 is a diagram showing an instrument distal end according to the embodiment.
FIG. 6 is a perspective view showing the configuration of an arm operation unit according to the embodiment.
FIG. 7 is a diagram for illustrating linear motion of the robot arm.
FIG. 8 is a diagram for illustrating rotational movement of the robot arm.
FIG. 9 is a diagram showing an endoscope.
FIG. 10 is a diagram showing a pivot position setting instrument.
FIG. 11 is a diagram showing operation units according to the embodiment.
FIG. 12 is a diagram showing a right-handed wrist according to the embodiment.
FIG. 13 is a diagram showing a left-handed wrist according to the embodiment.
FIG. 14 is a sectional view taken along a plane including A25 and A26 axes of a wrist.
FIG. 15 is a sectional view taken along a plane including A26 and A27 axes of the wrist.
FIG. 16 is a perspective view showing foot pedals according to the embodiment.
FIG. 17 is a control block diagram of a robotic surgical system according to the embodiment.
FIG. 18 is a control block diagram of the robot arm according to the embodiment.
FIG. 19 is a control block diagram of a positioner and the medial cart according to the embodiment.
FIG. 20 is a control block diagram of the operation unit according to the embodiment.
FIG. 21 is a schematic view of an instrument during surgery according to the embodiment.
FIG. 22 is a schematic view of a holder position after removal of an originally attached instrument according to the embodiment.
FIG. 23 is a schematic view showing a state in which the robot arm is being operated with a joystick according to the embodiment.
FIG. 24 is a schematic view showing a state in which a replacement instrument is attached according to the embodiment.
FIG. 25 is a schematic view showing a state in which the replacement instrument is moved to an inclination at which the originally attached instrument is removed according to the embodiment.
FIG. 26 is a schematic view after a guide tool change has been carried out according to the embodiment.
FIG. 27 is a control flow diagram of a surgical robot according to the embodiment.

### DESCRIPTION OF THE PREFERRED EMBODIMENTS

### Configuration of Robotic Surgical System

The configuration of a robotic surgical system 500 according to this embodiment is now described. The robotic surgical system 500 includes a surgical robot 100, a remote control apparatus 200, a vision unit 300, and an image processing unit 400. The remote control apparatus 200 is an example of an operation apparatus.

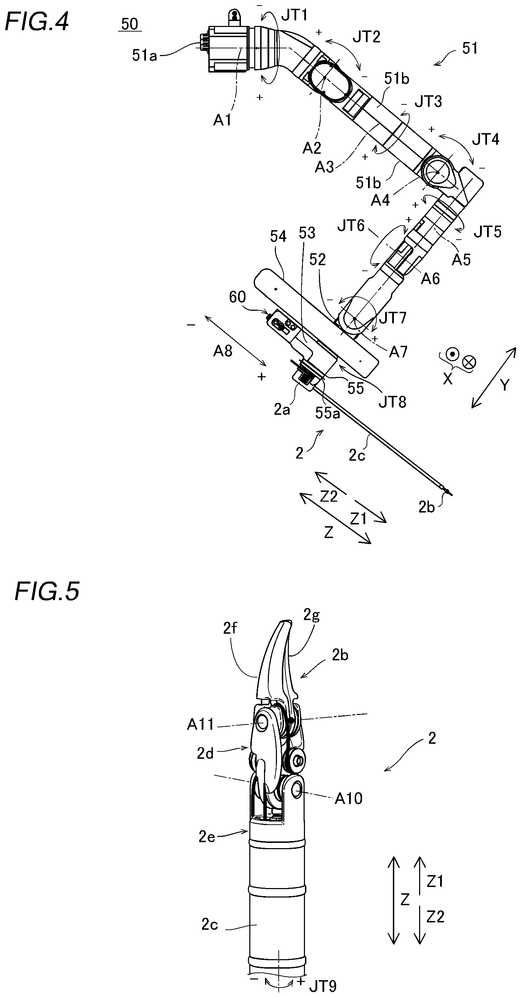

In this specification, as shown in FIG. 4, the longitudinal direction of an instrument 2 is defined as a Z direction. The distal end side of the instrument 2 is defined as a Z1 side, and the proximal end side of the instrument 2 is defined as a Z2 side. A direction perpendicular to the Z direction is defined as an X direction. A direction perpendicular to the Z direction and the X direction is defined as a Y direction.

In this specification, a direction perpendicular to a floor surface on which the remote control apparatus 200 is placed is defined as a Zb direction, the forward-rearward direction of an operator who operates operation units 110, which is perpendicular to the Zb direction, is defined as a Yb direction, and a direction perpendicular to the Zb direction and the Yb direction is defined as an Xb direction. In the Zb direction, an upward direction is defined as a Zb1 direction, and a downward direction is defined as a Zb2 direction. In the Yb direction, one side is defined as a Yb1 direction, and the other side is defined as a Yb2 direction. In the Xb direction, one side is defined as an Xb1 direction, and the other side is defined as an Xb2 direction. Moreover, the Xb direction, the Yb direction, and the Zb direction may be referred to as an Xb axis, a Yb axis, and a Zb axis, respectively.

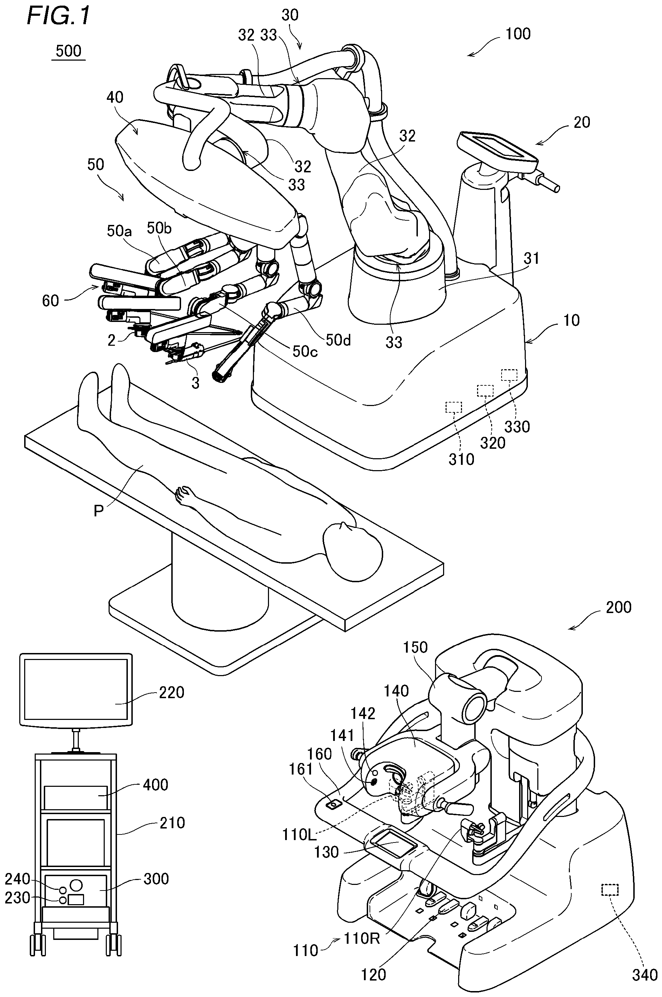

As shown in FIG. 1, the surgical robot 100 is arranged in an operating room. The remote control apparatus 200 is spaced apart from the surgical robot 100. The remote control apparatus 200 receives operations for the instrument 2. Specifically, an operator such as a doctor inputs a command to the remote control apparatus 200 to cause the surgical robot 100 to perform a desired operation. The remote control apparatus 200 transmits the input command to the surgical robot 100. The surgical robot 100 operates based on the received command. The surgical robot 100 is arranged in the operating room that is a sterilized sterile field.

### Configuration of Surgical Robot

As shown in FIG. 1, the surgical robot 100 includes a medical cart 10, a cart positioner operation unit 20, a positioner 30, an arm base 40, a plurality of robot arms 50, and an arm operation unit 60 provided on each of the robot arms 50.

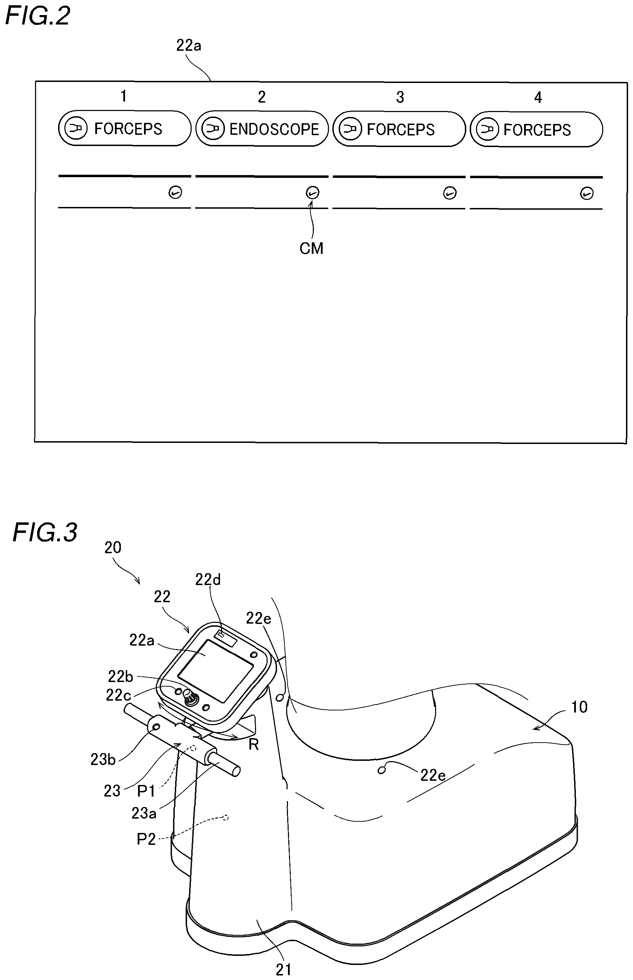

As shown in FIG. 3, the cart positioner operation unit 20 is supported by a cart positioner operation support 21 at the rear of the medical cart 10, and the medical cart 10 or the positioner 30 is moved by operating the cart positioner operation unit 20. The cart positioner operation unit 20 includes an input 22 and an operation handle 23. The input 22 receives operations to move the positioner 30, the arm base 40, and the plurality of robot arms 50 or change their postures mainly in order to prepare for surgery before the surgery. The medical cart 10 includes the operation handle 23.

As shown in FIG. 3, the input 22 includes a display 22a, a joystick 22b, an enable switch 22c, an error reset button 22d, and speakers 22e. The display 22a is a liquid crystal panel, for example. As shown in FIG. 2, the display 22a displays numbers corresponding to the plurality of robot arms 50. The display 22a also displays the type of instrument 2 attached to each of the plurality of robot arms 50. A check mark CM indicating that a pivot position PP described below has been set is displayed on the display 22a.

As shown in FIG. 3, the joystick 22b is arranged in the vicinity of or adjacent to the display 22a of the input 22 of the medical cart 10. The positioner 30 is moved three-dimensionally by selecting an operation mode displayed on the display 22a and operating the joystick 22b.

The enable switch 22c is arranged in the vicinity of or adjacent to the joystick 22b. The enable switch 22c enables or disables movement of the positioner 30. When the joystick 22b is operated while the enable switch 22c is pressed to enable movement of the positioner 30, the positioner 30 is moved.

The error reset button 22d resets errors in the robotic surgical system 500. The errors may be abnormal deviation errors, for example. The speakers 22e are arranged in pair. The pair of speakers 22e are arranged in the vicinity of or adjacent to the location of the positioner 30 in the medical cart 10.

The operation handle 23 is arranged in the vicinity of the display 22a. The operation handle 23 includes a throttle 23a that is gripped and twisted by an operator such as a nurse or a technician to operate movement of the medical cart 10. Specifically, the operation handle 23 is arranged below the input 22. As the throttle 23a is twisted from the near side to the far side, the medical cart 10 moves forward. As the throttle 23a is twisted from the far side to the near side, the medical cart 10 moves rearward. The speed of the medical cart 10 is changed according to a twisting amount of the throttle 23a. The operation handle 23 is rotatable to the left and right shown by an R direction, and the medical cart 10 is turned with rotation of the operation handle 23.

An enable switch 23b for enabling or disabling movement of the medical cart 10 is provided on the operation handle 23 of the cart positioner operation unit 20. When the throttle 23a of the operation handle 23 is operated while the enable switch 23b is pressed to enable movement of the medical cart 10, the medical cart 10 is moved.

As shown in FIG. 1, the positioner 30 includes a 7-axis articulated robot, for example. The positioner 30 is arranged on the medical cart 10. The positioner 30
adjusts the position of the arm base 40. The positioner 30 moves the position of the arm base 40 three-dimensionally.

The positioner 30 includes a base 31 and a plurality of links 32 coupled to the base 31. The plurality of links 32 are coupled to each other by joints 33.

The arm base 40 is attached to the distal end of the positioner 30. The proximal end of each of the plurality of robot arms 50 is attached to the arm base 40. Each of the plurality of robot arms 50 is able to take a folded and stored posture. The arm base 40 and the plurality of robot arms 50 are covered with sterile drapes and used. Moreover, each of the robot arms 50 supports the instrument 2.

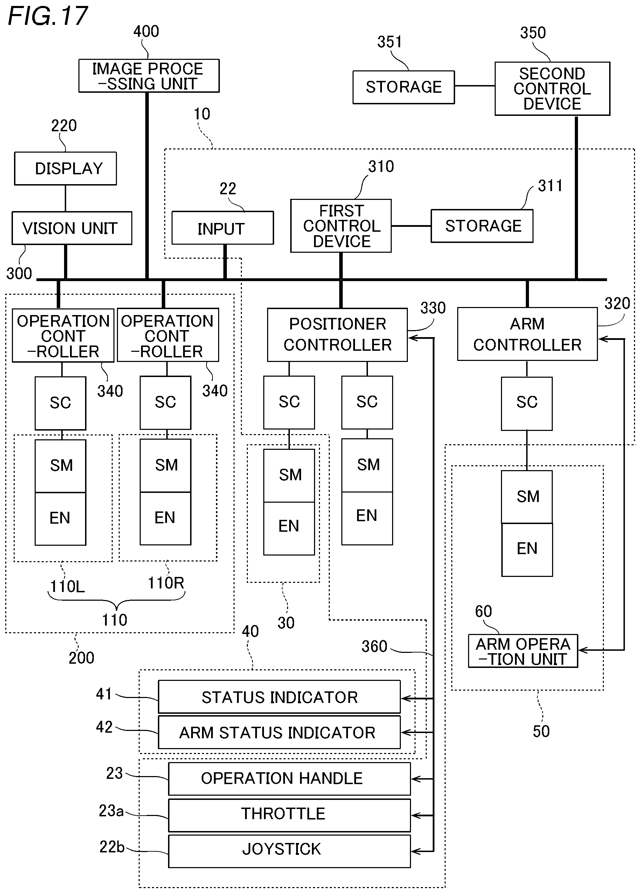

A status indicator 41 and an arm status indicator 42 that are shown in FIG. 17 are provided on the arm base 40. The status indicator 41 indicates the status of the robotic surgical system 500. The arm status indicator 42 indicates the statuses of the robot arms 50.

The plurality of robot arms 50 are arranged. Specifically, four robot arms 50a, 50b, 50c, and 50d are arranged. The robot arms 50a, 50b, 50c, and 50d have the same or similar configurations as each other.

As shown in FIG. 4, each robot arm 50 includes an arm portion 51, a first link 52, a second link 53, and a linear motion mechanism 54. The robot arm 50 includes joints JT1, JT2, JT3, JT4, JT5, JT6, JT7, and JT8. The joints JT1, JT2, JT3, JT4, JT5, JT6, and JT7 have A1, A2, A3, A4, A5, A6, and A7 axes as rotation axes, respectively. The joint JT8 has an A8 axis as a linear motion axis. The A1 to A7 axes are rotation axes from the joints JT1 to JT7 of the arm portion 51, respectively. The A7 axis is the rotation axis of the first link 52. The A8 axis is a linear motion axis along which the linear motion mechanism 54 moves the second link 53 relative to the first link 52 in the Z direction. The second link 53 is an example of a movable element. The arm portion 51 includes a base 51a and links 51b.

The arm portion 51 includes a 7-axis articulated robot arm. The first link 52 is arranged at the distal end of the arm portion 51. The arm operation unit 60 described below is attached to the second link 53. The linear motion mechanism 54 is arranged between the first link 52 and the second link 53. A holder 55 that holds the instrument 2 is arranged on the second link 53. The linear motion mechanism 54 linearly moves the holder 55 to which the instrument 2 is attached between a first position and a second position. The first position refers to an end position on the Z2 side in a range of movement of the holder 55 along the A8 axis by the linear motion mechanism 54. The second position refers to an end position on the Z1 side in the range of movement of the holder 55 along the A8 axis by the linear motion mechanism 54.

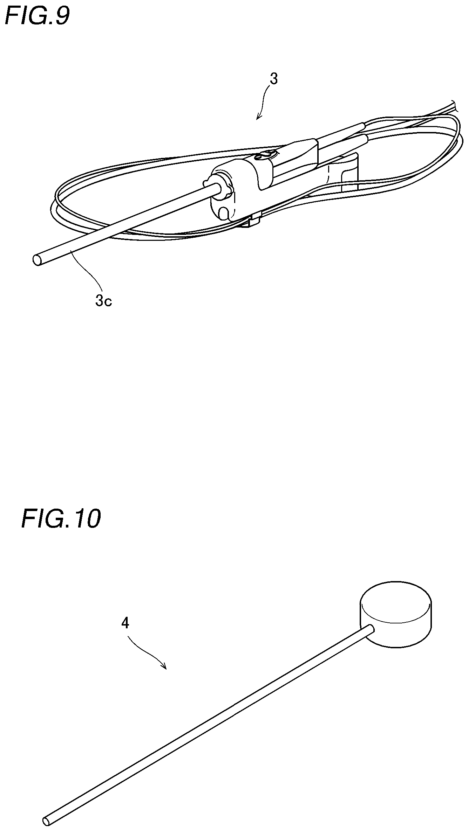

The instrument 2 is attached to the distal end of each of the plurality of robot arms 50. The instrument 2 includes a replaceable instrument 2, an endoscope 3 shown in FIG. 9 to capture an image of a surgical site, and a pivot position setting instrument 4 shown in FIG. 10 to set the pivot position PP, for example. The instrument 2 includes a driven unit 2a, an end effector 2b, and a shaft 2c.

As shown in FIG. 1, the endoscope 3 is attached to the distal end of one of the plurality of robot arms 50, such as the robot arm 50c, and the instrument 2 is attached to the distal end of each of the remaining robot arms 50a, 50b, and 50d, for example. The endoscope 3 is preferably attached to one of two robot arms 50b and 50c arranged in the center among the four robot arms 50 arranged adjacent to each other.

### Configuration of Instrument

As shown in FIG. 5, the end effector 2b is provided at the distal end of the instrument 2. As examples of the end effector 2b having joints, a pair of scissors, a grasper, a needle holder, a microdissector, a stable applier, a tacker, a suction cleaning tool, a snare wire, a clip applier, etc. are arranged. At the distal end of the instrument 2, a cutting blade, a cautery probe, a washer, a catheter, a suction orifice, etc. are arranged as instruments having no joint.

The end effector 2b includes a first support 2d and a second support 2e. The first support 2d supports the proximal end sides of jaw members 2f and 2g such that the jaw members 2f and 2g are rotatable around an A11 axis. The second support 2e supports the proximal end side of the first support 2d such that the first support 2d is rotatable around an A10 axis. The shaft 2c rotates around an A9 axis. The jaw members 2f and 2g pivot around the A11 axis to open and close. The second support 2e is connected to the shaft 2c. As the length of a portion of the instrument 2 that can be inserted into the body of a patient increases, the surgical procedure can be performed up to a position farther from a trocar T. For example, the length is about 500 mm. Even when the type of instrument 2 is different, the required length of insertion into the body is the same.

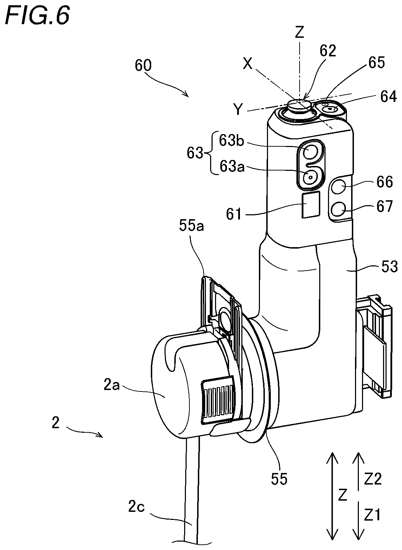

As shown in FIG. 6, the instrument 2 is engaged with the holder 55 via a drape adapter 55a. That is, the term "the holder 55 to which the instrument 2 is attached" indicates a broader concept including a case in which the instrument 2 is directly attached to the holder 55 and a case in which the instrument 2 is attached to the holder 55 via the drape adapter 55a. The drape adapter 55a is attached to the holder 55 when a drape is attached in preparation for surgery, and is not removed during surgery. When the instrument 2 is replaced, the instrument 2 is removed from the drape adapter 55a, and another instrument 2A is attached.

### Configuration of Arm Operation Unit

As shown in FIG. 6, the arm operation unit 60 is attached to the robot arm 50 to operate the robot arm 50. Specifically, the arm operation unit 60 is attached to the second link 53.

The arm operation unit 60 includes an enable switch 61, a joystick 62, and linear switches 63, a mode switching button 64, a mode indicator 65, a pivot button 66, and an adjustment button 67. The joystick 62 is an example of an operation tool.

The enable switch 61 is pressed to enable or disable movement of the robot arm 50 in response to the joystick 62 and the linear switches 63. When the enable switch 61 is pressed by an operator such as a nurse or an assistant grasping the arm operation unit 60, movement of the instrument 2 by the robot arm 50 is enabled. The enable switch 61 is an example of an input element.

The joystick 62 is an operation tool to control movement of the instrument 2 by the robot arm 50. The joystick 62 controls a moving direction and a moving speed of the robot arm 50. The robot arm 50 is moved in accordance with a tilting direction and a tilting angle of the joystick 62.

The linear switches 63 are switches to move the instrument 2 in the Z direction, which is the longitudinal direction of the instrument 2. The linear switches 63 include a linear switch 63a to move the instrument 2 in a direction in which the instrument 2 is inserted into a patient P, and a linear switch 63b to move the instrument 2 in a direction in which the instrument 2 is moved away from the patient P. Both the linear switch 63a and the linear switch 63b are push-button switches.

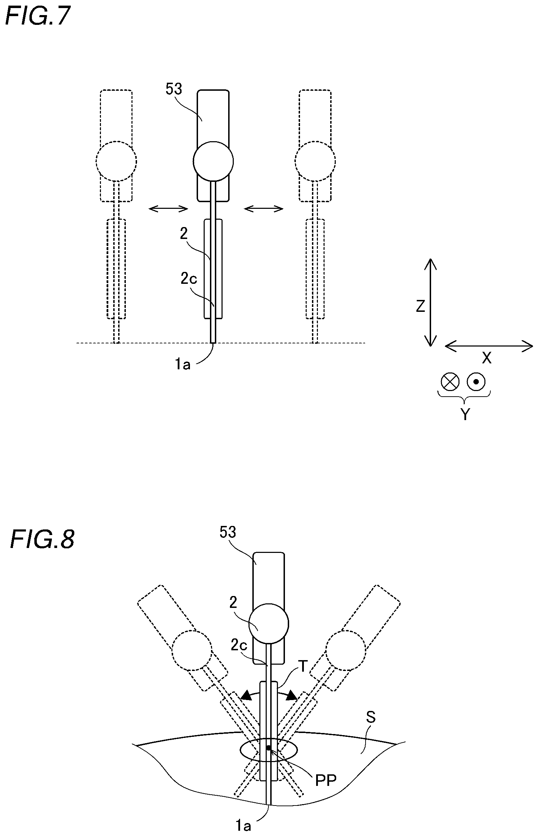

The mode switching button 64 is a push-button switch to switch between a mode for translationally moving the instrument 2 when not in surgery and a mode for rotationally moving the instrument 2. Examples of times other than during surgery include during preparation work before the start of surgery, during pivot position teaching work, and during work after the end of surgery. As shown in FIG. 7, in the mode for translationally moving the robot arm 50, the robot arm 50 is moved such that the distal end 1a of the instrument 2 is moved in an X-Y plane. As shown in FIG. 8, in the mode for rotationally moving the robot arm 50, the robot arm 50 is moved such that the instrument 2 is rotationally moved around a center on the A11 axis of the end effector 2b or the distal end of the end effector 2b of the instrument 2 when any pivot position PP is not stored in a storage 351, and the instrument 2 is rotationally moved around the pivot position PP as a fulcrum when the pivot position PP is stored in the storage 351. In this case, the instrument 2 is rotationally moved with the shaft 2c of the instrument 2 inserted into the trocar T. The mode switching button 64 is arranged on a Z-direction side surface of the arm operation unit 60.

The mode indicator 65 indicates a switched mode. The mode indicator 65 is on to indicate a rotational movement mode and is off to indicate a translational movement mode. Furthermore, the mode indicator 65 also serves as a pivot position indicator that indicates that the pivot position PP has been set. The mode indicator 65 is arranged on the Z-direction side surface of the arm operation unit 60.

The pivot button 66 is a push-button switch to set the pivot position PP that serves as a fulcrum for movement of the instrument 2 attached to the robot arm 50.

The adjustment button 67 is a button to optimize the position of the robot arm 50. After the pivot position PP for the robot arm 50 to which the endoscope 3 has been attached is set, the positions of the other robot arms 50 and the arm base 40 are optimized when the adjustment button 67 is pressed. The adjustment button 67 is a button different from the enable switch 61.

### Remote Control Apparatus

As shown in FIG. 1, the remote control apparatus 200 is arranged inside or outside the operating room, for example. The remote control apparatus 200 includes the operation units 110, foot pedals 120, a touch panel 130, a monitor 140, a support arm 150, a support bar 160, an error reset button 161. The operation units 110 include operation handles for the operator such as a doctor to input a command. The monitor 140 is a scope-type display that displays images captured by the endoscope 3. A notifier 141 and a head sensor 142 are provided on the monitor 140. The notifier 141 issues an error sound. The head sensor 142 detects whether or not the doctor who operates the remote control apparatus 200 is visually checking the monitor 140. The head sensor 142 is an optical sensor, for example. When the head of the doctor is present in the vicinity of the head sensor 142, the head sensor 142 detects that the doctor is visually checking the monitor 140. The support arm 150 supports the monitor 140 so as to align the height of the monitor 140 with the height of the face of the operator such as a doctor. The touch panel 130 is arranged on the support bar 160. The head of the operator is detected by the head sensor 142 provided in the vicinity of the monitor 140 such that the surgical robot 100 can be operated by the remote control apparatus 200. The operator operates the operation units 110 and the foot pedals 120 while visually recognizing an affected area on the monitor 140. Thus, a command is input to the remote control apparatus 200. The command input to the remote control apparatus 200 is transmitted to the surgical robot 100. The error reset button 161 is arranged on the support bar 160. The error reset button 161 resets errors in the robotic surgical system 500. The errors may be abnormal deviation errors, for example.

### Operation Unit

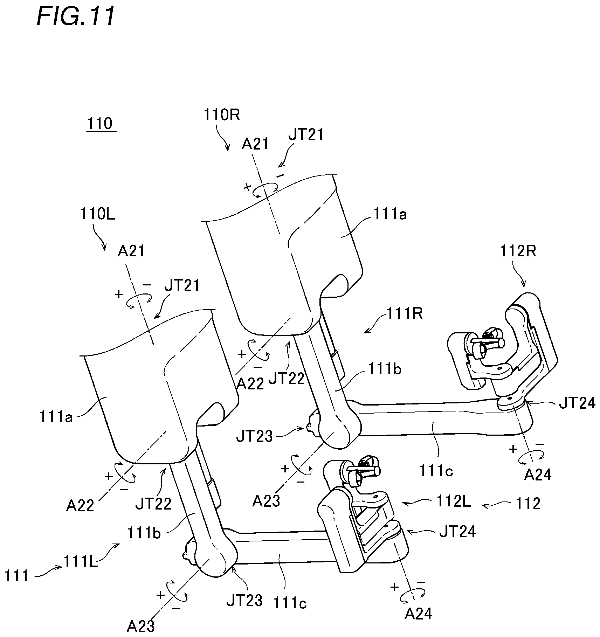

As shown in FIG. 11, the operation units 110 each include a handle to operate the instrument 2. The operation unit 110 receives an operation for the instrument 2. The operation units 110 include an operation unit 110L that is located on the left side as viewed from the operator such as a doctor and is to be operated by the left hand of the operator, and an operation unit 110R that is located on the right side and is to be operated by the right hand of the operator. The operation units 110 include arms 111 and wrists 112. The operation unit 110R includes an arm 111R and a wrist 112R. The operation unit 110L includes an arm 111L and a wrist 112L.

As shown in FIGS. 11, 12, and 13, the operation units 110 each have joints JT21, JT22, JT23, JT24, JT25, JT26, and JT27. The rotation axes of the joints JT21, JT22, JT23, JT24, JT25, JT26, and JT27 are A21, A22, A23, A24, A25, A26, and A27 axes, respectively.

As shown in FIG. 11, each of the arms 111 includes a link 111a, a link 111b, and a link 111c. The upper end side of the link 111a is attached to the remote control apparatus 200 such that the link 111a is rotatable around the A21 axis along a vertical direction. The upper end side of the link 111b is attached to the lower end side of the link 111a such that the link 111b is rotatable around the A22 axis along a horizontal direction. A first end side of the link 111c is attached to the lower end side of the link 111b such that the link 111c is rotatable around the A23 axis along the horizontal direction. The wrist 112 is attached to a second end side of the link 111c such that the wrist 112 is rotatable around the A24 axis. The link 111a is connected to the remote control apparatus 200 by the joint JT21. The link 111a and the link 111b are connected to each other by the joint JT22. The link 111b and the link 111c are connected to each other by the joint JT23. The arm 111 supports the wrist 112.

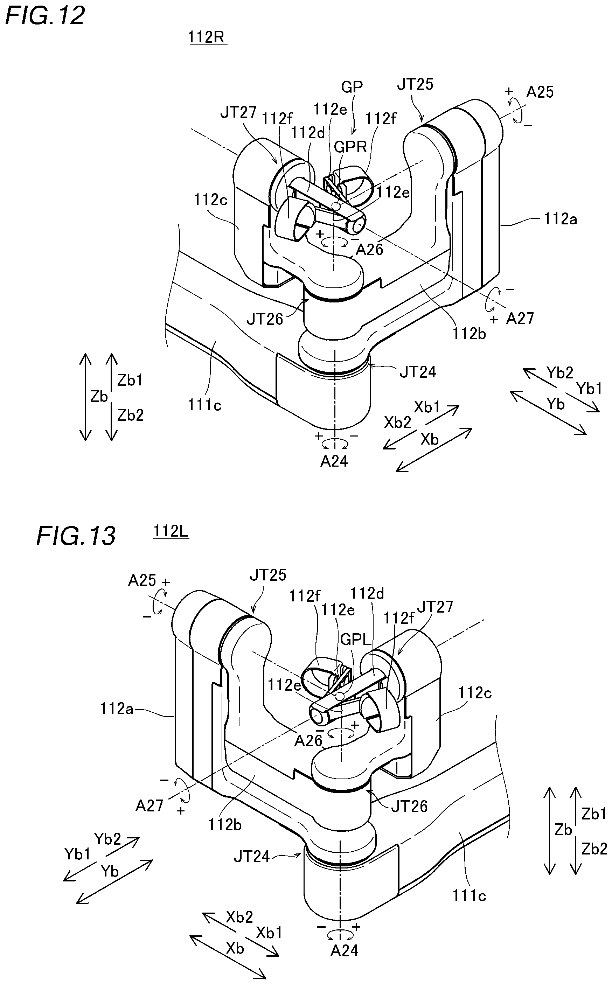

The wrists 112 include the wrist 112R shown in FIG. 12 and to be operated by the right hand of the operator, and the wrist 112L shown in FIG. 13 and to be operated by the left hand of the operator. FIG. 12 shows the reference posture of the operation unit 110R, and FIG. 13 shows the reference posture of the operation unit 110L. The configuration of the wrist 112R is the same as or similar to that of the wrist 112L.

A link 112a includes a proximal end connected to the distal end of the arm 111 and rotates around the A24 axis. A link 112b is attached to the link 112a so as to be rotatable around the A25 axis. A link 112c is attached to the link 112b so as to be rotatable around the A26 axis. A grip 112d is attached to the link 112c so as to be rotatable around the A27 axis. The link 112a, the link 112b, and the link 112c each have an L shape.

The wrist 112 includes a pair of grip members 112e that are opened and closed by the operator. The grip members 112e each include an elongated plate-shaped lever member, and the proximal ends of the pair of grip members 112e are rotatably connected to the proximal end of the grip 112d. Cylindrical finger insertion portions 112f are provided on the grip members 112e. The operator inserts their fingers into a pair of finger insertion portions 112f to operate the wrist 112. The proximal ends of the pair of grip members 112e are connected to the grip 112d, and an angle between the pair of grip members 112e is increased or decreased such that an opening angle between the jaw member 2f and the jaw member 2g is changed. A magnet is provided on one of the grip members 112e, and a Hall sensor is provided on the grip 112d. When the operator opens and closes the grip members 112e, the magnet and the Hall sensor function as an angle detection sensor, and the Hall sensor outputs the opening angle. As the angle detection sensor, the Hall sensor may be provided on the grip member 112e, and the magnet may be provided on the grip 112d. Alternatively, the magnet or the Hall sensor may be provided as the angle detection sensor on both the grip members 112e.

The intersection of a plurality of rotation axes of the operation unit 110 is called a gimbal point GP. Specifically, the gimbal point GP is a point at which the A24 axis, the A25 axis, the A26 axis, and the A27 axis intersect with each other. The gimbal point GP is located in the grip 112d to which the pair of grip members 112e are attached. The gimbal point GP exists individually in each of the operation unit 110L and the operation unit 110R. The gimbal point of the operation unit 110R is defined as GPR. The gimbal point of the operation unit 110L is defined as GPL.

In the reference posture, the A24 and A26 axes of the operation unit 110 are along the Zb direction. The A25 axis is along the Xb direction. The A27 axis is along the Yb direction. As shown in FIG. 12, in the reference posture, the link 112a and the link 112b of the wrist 112R are arranged along an Xb-Zb plane and on the Xb1 side with respect to the A27 axis. In the reference posture, the link 112c is arranged along a Yb-Zb plane. In the reference posture, the grip 112d is arranged along the A27 axis.

As shown in FIG. 13, in the reference posture, the link 112a and the link 112b of the wrist 112L are arranged along the Xb-Zb plane and on the Xb2 side with respect to the A27 axis. In the reference posture, the link 112c is arranged along the Yb-Zb plane. In the reference posture, the grip 112d is arranged along the A27 axis.

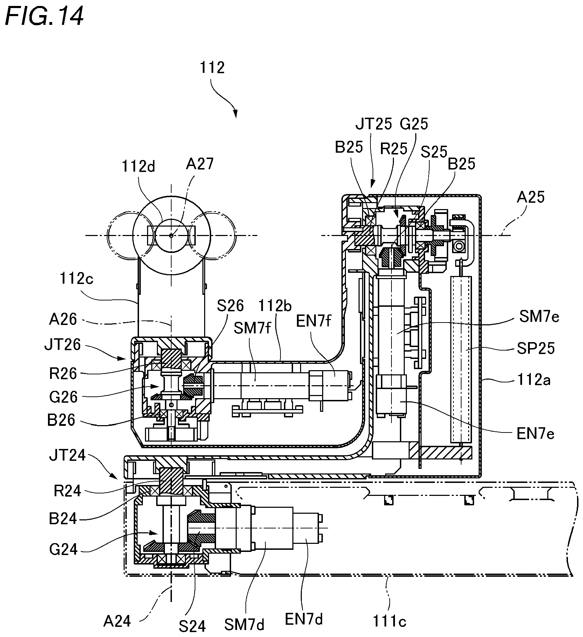

As shown in FIG. 14, the link 112a includes an elbow-shaped (L-shaped) box body, and accommodates main elements of the link 112a in the box body. A rotation shaft R24 is arranged at a first end of the link 112a. The rotation shaft R24 is attached to a second end of the link 111c via a bearing B24 so as to be rotatable around the A24 axis. The joint JT24 is defined by the rotation shaft R24 and the bearing B24. Thus, the link 112a can rotate around the A24 axis with respect to the link 111c.

A servomotor SM7d is arranged in the link 111c such that the central axis of a main shaft S24 is perpendicular to the A24 axis. An encoder EN7d is provided on the servomotor SM7d to detect the rotation angle of the servomotor SM7d. Any encoder that can detect the rotation angle can be used as the encoder EN7d, or a tachometer or the like may be used instead of the encoder EN7d. The encoder EN7d is directly coupled to the main shaft S24 of the servomotor SM7d. The main shaft S24 of the servomotor SM7d is connected to the rotation shaft R24 via a bevel gear mechanism G24. Thus, the encoder EN7d can detect the rotation angle of the servomotor SM7d when the link 112a rotates, and the rotation shaft R24 can be rotatably driven by the servomotor SM7d.

The link 112b includes an elbow-shaped (L-shaped) box body, and accommodates main elements of the link 112b in the box body. A rotation shaft R25 is arranged at a first end of the link 112b. The rotation shaft R25 is attached to a second end of the link 112a via a bearing B25 so as to be rotatable around the A25 axis. The joint JT25 is defined by the rotation shaft R25 and the bearing B25. Thus, the link 112b can rotate around the A25 axis with respect to the link 112a.

A servomotor SM7e is arranged in the link 112a such that the central axis of a main shaft S25 is perpendicular to the A25 axis. An encoder EN7e is provided on the servomotor SM7e to detect the rotation angle of the servomotor SM7e. A tachometer or the like may be used instead of the encoder EN7e. The encoder EN7e is directly coupled to the main shaft S25 of the servomotor SM7e. The main shaft S25 of the servomotor SM7e is connected to the rotation shaft R25 via a bevel gear mechanism G25. Thus, the encoder EN7e can detect the rotation angle of the servomotor SM7e when the link 112b rotates, and the rotation shaft R25 can be rotatably driven by the servomotor SM7e.

A compression coil spring SP25 is arranged between a predetermined portion of the link 112a and the rotation shaft R25. For example, the predetermined portion is a lower end of a rear end of the link 112a in the reference posture of the wrist 112. The compression coil spring SP25 is arranged with its central axis being parallel to the A24 axis and perpendicular to the A25 axis. Furthermore, the compression coil spring SP25 is designed such that a predetermined torque is applied to the link 112b in a rotation direction when the link 112b rotates from the reference posture of the wrist 112. The predetermined torque is predetermined to partially counteract a gravitational torque generated on the rotation shaft R25 by the self-weight of the link 112b and the self-weight of a portion of the wrist 112 on the distal side with respect to the link 112b. Thus, a portion of the gravitational torque generated on the rotation shaft R25 is canceled by the compression coil spring SP25.

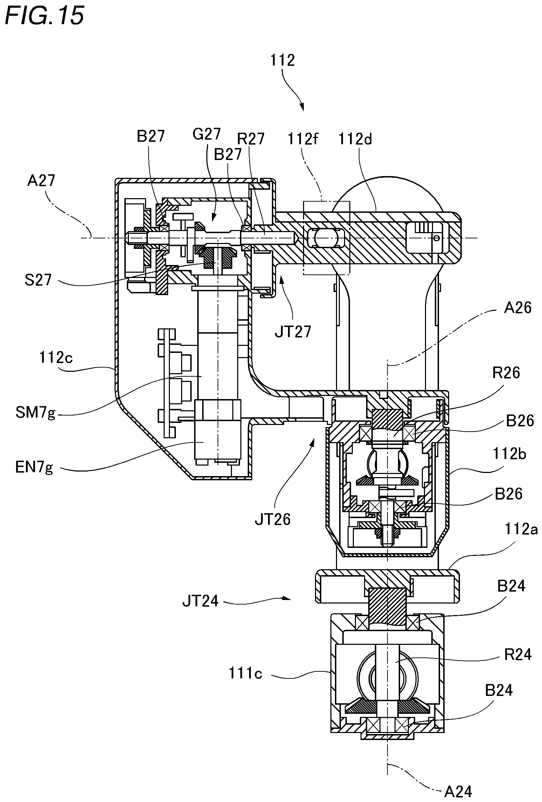

As shown in FIGS. 14 and 15, the link 112c includes an elbow-shaped (L-shaped) box body, and accommodates main elements of the link 112c in the box body. A rotation shaft R26 is arranged at a first end of the link 112c. The rotation shaft R26 is attached to a second end of the link 112b via a bearing B26 so as to be rotatable around the A26 axis. The joint JT26 is defined by the rotation shaft R26 and the bearing B26. Thus, the link 112c can rotate around the A26 axis with respect to the link 112b.

A servomotor SM7f is arranged in the link 112b such that the central axis of a main shaft S26 is perpendicular to the A26 axis. An encoder EN7f is provided on the servomotor SM7f to detect the rotation angle of the servomotor SM7f. A tachometer or the like may be used instead of the encoder EN7f. The encoder EN7f is directly coupled to the main shaft S26 of the servomotor SM7f. The main shaft S26 of the servomotor SM7f is connected to the rotation shaft R26 via a bevel gear mechanism G26. Thus, the encoder EN7f can detect the rotation angle of the servomotor SM7f when the link 112c rotates, and the rotation shaft R26 can be rotatably driven by the servomotor SM7f.

As shown in FIG. 15, a rotation shaft R27 is arranged at a first end of the grip 112d. The rotation shaft R27 is attached to a second end of the link 112c via a bearing B27 so as to be rotatable around the A27 axis. The joint JT27 is defined by the rotation shaft R27 and the bearing B27. Thus, the grip 112d can rotate around the A27 axis with respect to the link 112c.

A servomotor SM7g is arranged in the link 112c such that the central axis of a main shaft S27 is perpendicular to the A27 axis. An encoder EN7g is provided in the servomotor SM7g to detect the rotation angle of the servomotor SM7g. A tachometer or the like may be used instead of the encoder EN7g. The encoder EN7g is directly coupled to the main shaft S27 of the servomotor SM7g. The main shaft S27 of the servomotor SM7g is connected to the rotation shaft R27 via a bevel gear mechanism G27. Thus, the encoder EN7g can detect the rotation angle of the servomotor SM7g when the grip 112d rotates, and the rotation shaft R27 can be rotatably driven by the servomotor SM7g.

### Foot Pedals

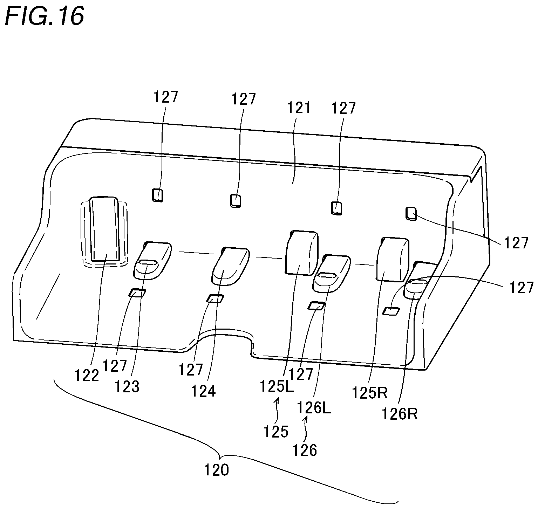

As shown in FIG. 16, a plurality of foot pedals 120 are provided to perform functions related to the instrument 2. The plurality of foot pedals 120 are arranged on a base 121. The foot pedals 120 include a switching pedal 122, a clutch pedal 123, a camera pedal 124, incision pedals 125, coagulation pedals 126, and foot detectors 127. The switching pedal 122, the clutch pedal 123, the camera pedal 124, the incision pedals 125, and the coagulation pedals 126 are operated by the foot of the operator. The incision pedals 125 include an incision pedal 125R for a right robot arm 50, and an incision pedal 125L for a left robot arm 50. The coagulation pedals 126 include a coagulation pedal 126R for the right robot arm 50 and a coagulation pedal 126L for the left robot arm 50.

The switching pedal 122 switches robot arms 50 to be operated by the operation units 110. The clutch pedal 123 performs a clutch operation to temporarily disconnect an operation connection between the robot arms 50 and the operation units 110. While the clutch pedal 123 is being pressed by the operator, operations by the operation units 110 are not transmitted to the robot arms 50. While the camera pedal 124 is being pressed by the operator, the operation unit 110 can operate a robot arm 50 to which the endoscope 3 is attached. While the incision pedals 125 or the coagulation pedals 126 are being pressed by the operator, an electrosurgical device is activated.

The foot detectors 127 detect the foot of the operator that operates the foot pedals 120. The foot detectors 127 are provided for the switching pedal 122, the clutch pedal 123, the camera pedal 124, the incision pedal 125L, the coagulation pedal 126L, the incision pedal 125R, and the coagulation pedal 126R, and detect the foot that hovers above their corresponding foot pedals 120. The foot detectors 127 are arranged on the base 121. The functions of the foot pedals 120 including the camera pedal 124 are not limited to being performed through pedals configured to be pressed by the foot of the operator as in this embodiment, but inputs such as hand switches may be provided on the operation units 110, and manual operations by the operator may be used, for example.

### Vision Unit and Image Processing Unit

As shown in FIG. 1, the vision unit 300 and the image processing unit 400 are placed on a cart 210. The image processing unit 400 processes images captured by the endoscope 3. A display 220 is arranged on the cart 210. The display 220 displays images captured by the endoscope 3. An error reset button 230 and a notifier 240 are arranged on the vision unit 300. The error reset button 230 resets errors in the robotic surgical system 500. The errors may be abnormal deviation errors, for example. The notifier 240 issues an error sound.

### Configuration of Control System

As shown in FIG. 17, the robotic surgical system 500 includes a first control device 310, an arm controller 320, a positioner controller 330, operation controllers 340, and a second control device 350. The robotic surgical system 500 also includes a storage 311 connected to the first control device 310 and the storage 351 connected to the second control device 350. The first control device 310 is an example of a controller.

The first control device 310 is accommodated in the medical cart 10 to communicate with the arm controller 320 and the positioner controller 330, and controls the entire robotic surgical system 500. Specifically, the first control device 310 communicates with and controls the arm controller 320, the positioner controller 330, and the operation controllers 340. The first control device 310 is connected to the arm controller 320, the positioner controller 330, and the operation controllers 340 through a LAN, for example. The first control device 310, the storage 311 described below, the second control device 350, and the storage 351 are arranged inside the medical cart 10 of the surgical robot 100.

The arm controller 320 is arranged for each of the plurality of robot arms 50. That is, the same number of arm controllers 320 as the plurality of robot arms 50 are placed inside the medical cart 10.

As shown in FIG. 17, the input 22 is connected to the first control device 310 through a LAN, for example. The status indicator 41, the arm status indicator 42, the operation handle 23, the throttle 23a, and the joystick 22b are connected to the positioner controller 330 via a wire line 360 by means of a communication network that allows information to be shared with each other by using serial communication. Although FIG. 17 shows that the status indicator 41, the arm status indicator 42, etc. are all connected to one wire line 360, in reality, the wire line 360 is arranged for each of the status indicator 41, the arm status indicator 42, the operation handle 23, the throttle 23a, and the joystick 22b.

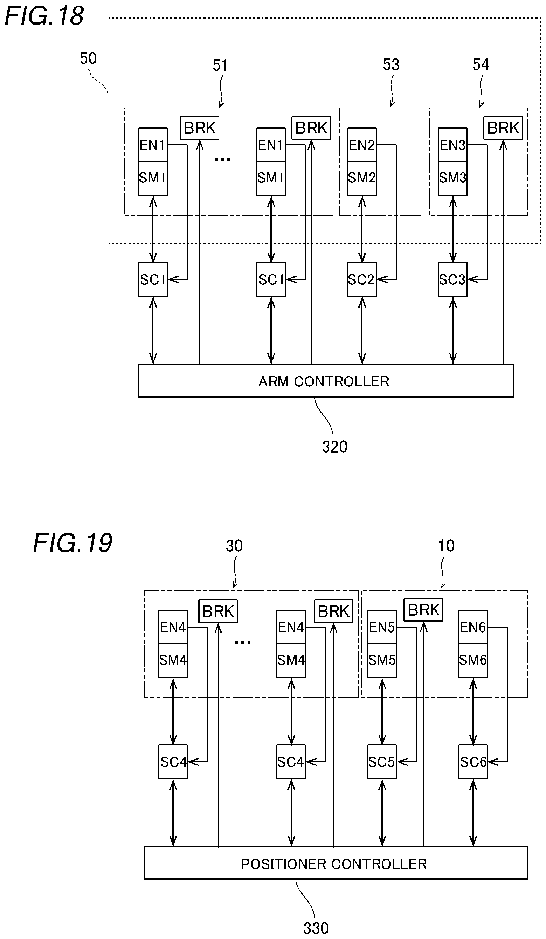

As shown in FIG. 18, the arm portion 51 includes a plurality of servomotors SM1, encoders EN1, and speed reducers so as to correspond to the joints JT1, JT2, JT3, JT4, JT5, JT6, and JT7. The encoders EN1 detect rotation angles of the servomotors SM1. The speed reducers slow down rotation of the servomotors SM1 to increase the torques. Inside the medical cart 10, servo controllers SC1 that control the servomotors SM1 are arranged adjacent to the arm controller 320. In addition, the encoders EN1 that detect the rotation angles of the servomotors SM1 are electrically connected to the servo controllers SC1.

Servomotors SM2 that rotate driven members provided in the driven unit 2a of the instrument 2, encoders EN2, and speed reducers are arranged in the second link 53. The encoders EN2 detect rotation angles of the servomotors SM2. The speed reducers slow down rotation of the servomotors SM2 to increase the torques. In the medical cart 10, servo controllers SC2 are provided to control the servomotors SM2 to drive the instrument 2. The encoders EN2 that detect the rotation angles of the servomotors SM2 are electrically connected to the servo controllers SC2. A plurality of servomotors SM2, a plurality of encoders EN2, and a plurality of servo controllers SC2 are arranged.

The linear motion mechanism 54 includes a servomotor SM3 to linearly move the instrument 2, an encoder EN3, and a speed reducer. The encoder EN3 detects a rotation angle of the servomotor SM3. The speed reducer slows down rotation of the servomotor SM3 to increase the torque. In the medical cart 10, a servo controller SC3 is provided to control the servomotor SM3 to linearly move the instrument 2. The encoder EN3 that detects the rotation angle of the servomotor SM3 is electrically connected to the servo controller SC3.

The first control device 310 generates command values to command the positions of the servomotors SM1, SM2, and SM3 based on operations received by the remote control apparatus 200, and drives the servomotors SM1, SM2, and SM3 based on the command values. The first control device 310 detects an abnormal deviation error when differences between the command values and the positions of the servomotors SM1, SM2, and SM3 detected by sensors exceed an allowable range.

As shown in FIG. 19, a plurality of servomotors SM4, a plurality of encoders EN4, and a plurality of speed reducers are provided in the positioner 30 so as to correspond to a plurality of joints 33 of the positioner 30. The encoders EN4 detect rotation angles of the servomotors SM4. The speed reducers slow down rotation of the servomotors SM4 to increase the torques.

The medical cart 10 includes wheels including front wheels as drive wheels and rear wheels that are steered by the operation handle 23. The rear wheels are arranged closer to the operation handle 23 than the front wheels. The medical cart 10 includes servomotors SM5 to drive a plurality of front wheels of the medical cart 10, encoders EN5, speed reducers, and brakes BRK. The speed reducers slow down rotation of the servomotors SM5 to increase the torques. A potentiometer P1 shown in FIG. 3 is provided on the operation handle 23 of the medical cart 10, and the servomotors SM5 of the front wheels are driven based on a rotation angle detected by the potentiometer P1 according to the twist of the throttle 23a. Rear wheels of the medical cart 10 are of the dual wheel type, and the rear wheels are steered based on rightward-leftward rotation of the operation handle 23. Furthermore, a potentiometer P2 shown in FIG. 3 is provided on a rotation axis of the operation handle 23 of the medical cart 10, and servomotors SM6, encoders EN6, and speed reducers are provided on the rear wheels of the medical cart 10. The speed reducers slow down rotation of the servomotors SM6 to increase the torques. The servomotors SM6 are driven based on a rotation angle detected by the potentiometer P2 according to rightward-leftward rotation of the operation handle 23. That is, steering of the rear wheels by the rightward-leftward rotation of the operation handle 23 is power-assisted by the servomotors SM6.

The front wheels of the medical cart 10 are driven such that the medical cart 10 moves in a forward-rearward direction. Furthermore, the operation handle 23 of the medical cart 10 is rotated such that the rear wheels are steered, and the medical cart 10 turns in a right-left direction.

As shown in FIG. 19, in the medical cart 10, servo controllers SC4 are provided to control the servomotors SM4 to move the positioner 30. The encoders EN4 that detect the rotation angles of the servomotors SM4 are electrically connected to the servo controllers SC4. In the medical cart 10, servo controllers SC5 are provided to control the servomotors SM5 to drive the front wheels of the medical cart 10. The encoders EN5 that detect the rotation angles of the servomotors SM5 are electrically connected to the servo controllers SC5. In the medical cart 10, servo controllers SC6 are provided to control the servomotors SM6 to power-assist steering of the rear wheels of the medical cart 10. The encoders EN6 that detect the rotation angles of the servomotors SM6 are electrically connected to the servo controllers SC6.

As shown in FIGS. 18 and 19, the joints JT1, JT2, JT3, JT4, JT5, JT6, and JT7 of the arm portion 51, and the joints 33 of the positioner 30 include brakes BRK. Furthermore, the front wheels of the medical cart 10, the arm base 40, and the linear motion mechanism 54 include brakes BRK. The arm controller 320 unidirectionally transmits control signals to the brakes BRK of the joints JT1, JT2, JT3, JT4, JT5, JT6, and JT7 of the arm portion 51, and the linear motion mechanism 54. The control signals are signals for turning on/off the brakes BRK. The signals for turning on the brakes BRK include signals for maintaining the brakes BRK in an enabled state. The same applies to control signals from the positioner controller 330 to the brakes BRK of the joints 33 of the positioner 30 and the arm base 40. On startup, all the brakes BRK of the arm base 40, the arm portion 51, and the linear motion mechanism 54 are turned off but the servomotors SM are driven against gravity to maintain the postures of the robot arm 50 and the arm base 40. When an error occurs in the robotic surgical system 500, the brakes BRK of the arm base 40, the arm portion 51 and the linear motion mechanism 54 are turned on. When the error in the robotic surgical system 500 is reset, the brakes BRK of the arm base 40, the arm portion 51, and the linear motion mechanism 54 are turned off. When a shutdown operation is performed in the robotic surgical system 500, the brakes BRK of the arm base 40, the arm portion 51, and the linear motion mechanism 54 are turned on. The brakes BRK of the front wheels of the medical cart 10 are constantly turned on, and the brakes BRK are deactivated only while the enable switch 23b of the medical cart 10 is being pressed. The brakes BRK of the joints 33 of the positioner 30 are constantly turned on, and the brakes BRK are deactivated only while the enable switch 22c of the medical cart 10 is being pressed.

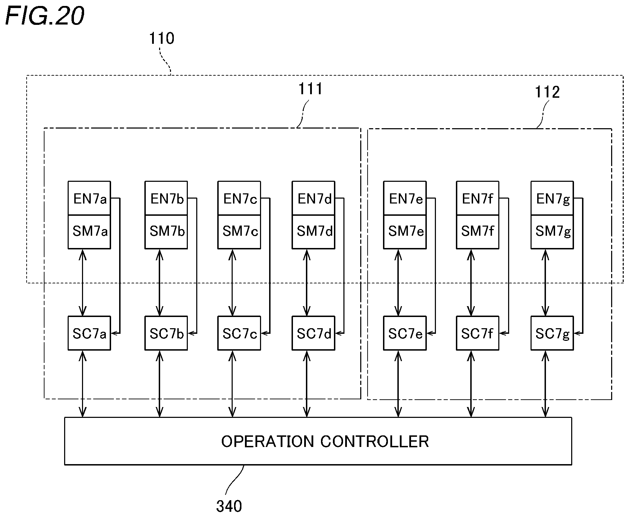

As shown in FIG. 20, servomotors SM7a, SM7b, SM7c, SM7d, SM7e, SM7f, and SM7g are arranged on the joints JT21, JT22, JT23, JT24, JT25, JT26, and JT27 of the operation unit 110, respectively. Servo controllers SC7a, SC7b, SC7c, SC7d, SC7e, SC7f, and SC7g are provided to control the servomotors. Encoders EN7a, EN7b, EN7c, EN7d, EN7e, EN7f, and EN7g are electrically connected to the servo controllers to detect rotation angles of the servomotors. The servomotors, the servo controllers, and the encoders are provided in each of the operation unit 110L and the operation unit 110R.

The first control device 310 controls the servomotors via the operation controllers 340 to generate torques that counteract gravitational torques generated on rotation axes of the servomotors according to the postures of the operation units 110. Thus, the operator can operate the operation units 110 with a relatively small force.

The first control device 310 generates torques on the rotation axes of the servomotors according to operations on the operation units 110 via the operation controllers 340, and controls the servomotors to assist the operation of the operator. Thus, the operator can operate the operation units 110 with a relatively small force.

As shown in FIG. 17, the first control device 310 controls the robot arm 50 based on an operation received by the arm operation unit 60. For example, the first control device 310 controls the robot arm 50 based on an operation received by the joystick 62 of the arm operation unit 60. Specifically, the arm controller 320 outputs an input signal input from the joystick 62 to the first control device 310. The first control device 310 generates position commands based on the received input signal and the rotation angles detected by the encoders EN1, and outputs the position commands to the servo controllers SC1 via the arm controller 320. The servo controllers SC1 generate current commands based on the position commands input from the arm controller 320 and the rotation angles detected by the encoders EN1, and output the current commands to the servomotors SM1. Thus, the robot arm 50 is moved according to an operation command input to the joystick 62.

The first control device 310 controls the robot arm 50 based on an input signal from either linear switch 63 of the arm operation unit 60. Specifically, the arm controller 320 outputs the input signal input from the linear switch 63 to the first control device 310. The first control device 310 generates a position command(s) based on the received input signal and the rotation angle(s) detected by the encoders EN1 or the encoder EN3, and outputs the position command(s) to the servo controllers SC1 or the servo controller SC3 via the arm controller 320. The servo controllers SC1 or the servo controller SC3 generates a current command(s) based on the position command(s) input from the arm controller 320 and the rotation angle(s) detected by the encoders EN1 or the encoder EN3, and outputs the current command(s) to the servomotors SM1 or the servomotor SM3. Thus, the robot arm 50 is moved according to an operation command input to the linear switch 63.

The positioner controller 330 is arranged in the medical cart 10. The positioner controller 330 controls the positioner 30 and the medical cart 10. The servomotors SM4, the encoders EN4, and the speed reducers are provided in the positioner 30 so as to correspond to the plurality of joints 33 of the positioner 30. The servo controllers SC4 are provided in the medical cart 10 to control the servomotors SM4 of the positioner 30. The servomotors SM5 and SM6 that drive the plurality of front wheels of the medical cart 10, the encoders EN5 and EN6, the speed reducers, the servo controllers SC5 and SC6, and the brakes BRK are provided in the medical cart 10.

The operation controllers 340 are arranged in a main body of the remote control apparatus 200. The operation controllers 340 control the operation units 110. The operation controllers 340 are provided so as to correspond to the left-handed operation unit 110L and the right-handed operation unit 110R, respectively, as shown in FIG. 17. Servomotors SM, encoders EN, and speed reducers are provided in the operation units 110 so as to correspond to a plurality of joints JT21 to JT27 of the operation units 110. Servo controllers SC that control the servomotors SM of the operation units 110 are provided adjacent to the operation controllers 340 in the main body of the remote control apparatus 200.

### Instrument Replacement

The replacement of the instrument 2 during surgery in this embodiment is now described. The instrument 2 has a shape and a function suitable for each purpose. For example, each purpose is grasping, dissection, ligation, suturing, etc. of the tissue of the patient, and in the case of an electric scalpel, it is incision, coagulation, etc. of the tissue using high-frequency current, but is not limited to these. The required instrument 2 changes depending on the progress of the surgery, and thus it is necessary to replace the instrument 2 attached to the holder 55 of the robot arm 50 with an instrument 2A suitable for the purpose at that time and continue the surgery. In order to continue the surgery, it is necessary to easily and quickly return the distal end position of the instrument 2A after replacement to the vicinity of the distal end position of the instrument 2 before replacement. The instrument 2 is an example of a first instrument, and the instrument 2A described below is an example of a second instrument.

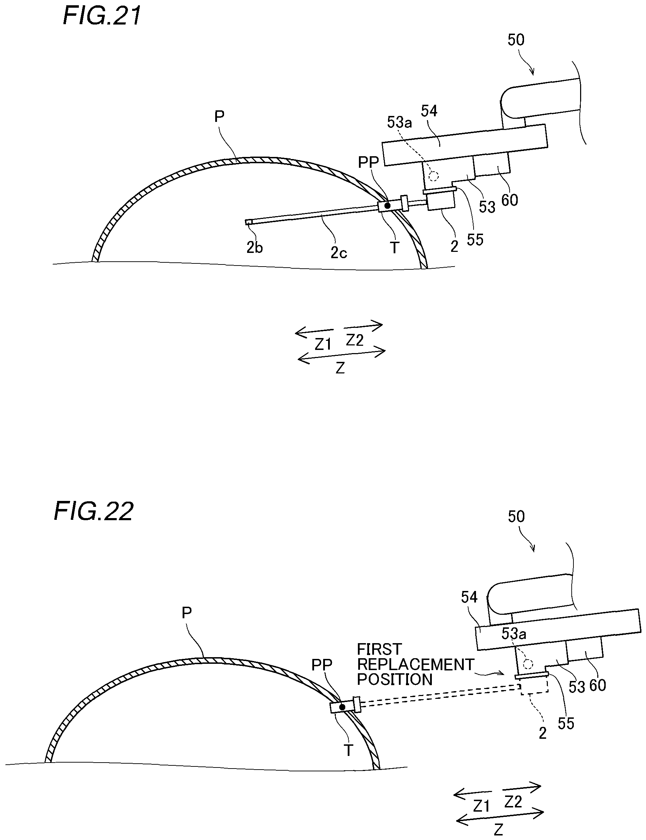

FIG. 21 is a schematic view showing a state in which surgery is performed using the instrument 2. The instrument 2 is attached to the holder 55 arranged at the distal end of the robot arm 50. The shaft 2c of the instrument 2 is inserted into the body of the patient P through the trocar T, and the end effector 2b arranged at the distal end of the instrument 2 is present inside the body of the patient P. When the operator operates the operation unit 110, the instrument 2 moves to a position and posture corresponding to the position and posture of the operation unit 110, but since the robot arm 50 is controlled such that the shaft 2c always passes through the pivot position PP, the abdominal wall of the patient P is not pulled by the shaft 2c, and the abdominal wall of the patient P is not damaged by the shaft 2c.

FIG. 22 is a schematic view with the instrument 2 removed for replacement. In the state of FIG. 21, in this embodiment, when the instrument 2 is removed from the holder 55, a detector 53a described below detects that the instrument 2 during surgery has been removed. That is, the detector 53a detects attachment and detachment of the instrument 2 to and from the holder 55. The storage 351 then stores the distal end position of the instrument 2 and the inclination of the shaft 2c. When the first support 2d or the jaw members 2f and 2g are inclined with respect to the longitudinal direction of the shaft 2c when the instrument 2 is removed, the storage 351 stores a position projected on an extension of the shaft 2c as the distal end position of the instrument 2. At the same time, the first control device 310 moves the holder 55 to a first replacement position when the detector 53a detects that the instrument 2 has been removed. Furthermore, when the detector 53a detects that the instrument 2 has been removed, the first control device 310 causes the linear motion mechanism 54 to move the holder 55 to a position that is a certain distance away from the pivot position PP. Specifically, when the instrument 2 is removed from the holder 55, the linear motion mechanism 54 of the robot arm 50 automatically moves to the first replacement position in a Z2 direction along the A8 axis. At this time, the instrument 2 is not attached to the holder 55, but when it is assumed that the instrument 2 is attached to the holder 55, the holder 55 and the trocar T are in a positional relationship in which the distal end of the instrument 2 is located inside the trocar T in the Z direction. The above is described in more detail. During the surgical operation, the robot arm 50 operates such that the end effector 2b at the distal end of the instrument 2 takes a desired position and posture by the combined operation of all axes from the A1 axis to the A8 axis. At this time, the first control device 310 controls the combined operation of the A1 axis to the A8 axis of the robot arm 50 such that the instrument 2 can always be moved into the trocar T by the operation of the linear motion mechanism 54 alone, regardless of the position and posture of the instrument 2 when it is replaced with another instrument 2A. When the robot arm 50 is used to cause the instrument 2 to take an arbitrary position and posture, six degrees of freedom of the axes of the robot arm 50 are sufficient. In other words, the robot arm 50 has three redundant degrees of freedom in order to change the position and posture of the instrument 2. The first control device 310 constantly monitors the positional relationship between the pivot position PP and the distal end of the instrument 2 during surgery. From the positional relationship, a distance required for the distal end of the instrument 2 to move into the trocar T is calculated, and the position of the second link 53 with respect to the linear motion mechanism 54 is determined based on the calculated distance. This is to determine the position of the A8 axis. Then, the A1 to A7 and A9 axes are controlled such that the instrument 2 takes a position and posture based on an input of the operation unit 110 with respect to the determined A8 axis. Therefore, when the instrument 2 is removed in an arbitrary position and posture and the linear motion mechanism 54 moves to the first replacement position in order to replace the instrument 2, the holder 55 is in a positional relationship in which the distal end of the instrument 2 would be located inside the trocar T if the instrument 2 were attached. The storage 351 stores at least information regarding the posture of the instrument 2 when the instrument 2 is removed. Specifically, the inclination of the shaft 2c of the instrument 2 when the instrument 2 is removed is stored in the storage 351. Information on the pivot position PP is also stored in the storage 351. The pivot position PP is a position set by the pivot button 66.

When the instrument 2 is replaced during surgery, the doctor basically checks the monitor 140 visually, and the head sensor 142 detects the head of the doctor. In this state, the robot arm 50 having the instrument 2 to be replaced cannot be operated by the operation unit 110, but the robot arms 50 holding other instruments 2 and the endoscope 3 can still be operated by the operation units 110. When the doctor moves away from the monitor 140 and the head sensor 142 does not detect the head of the doctor, all of the robot arms 50 cannot be operated by the operation units 110.

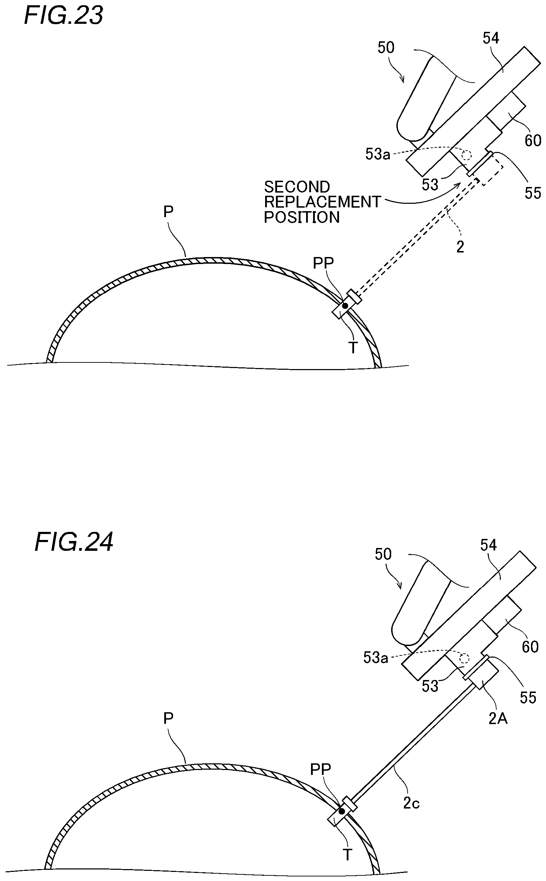

FIG. 23 is a schematic view of the robot arm 50 that a surgical staff operates with the joystick 62. The surgical staff is an assistant doctor or a nurse working on the patient P side, for example. In this embodiment, the first control device performs a control to move the holder 55 from the first replacement position to a second replacement position based on an input to the joystick 62. That is, when the instrument 2 is removed from the holder 55, the first control device 310 performs a control to enable the robot arm 50 to operate based on an operation input to the joystick 62 based on the detection of the removal of the instrument 2. Thus, the surgical staff can move the linear motion mechanism 54 of the robot arm 50 to a posture in which the instrument 2A can be easily attached by operating the joystick 62. In order to detect that the instrument 2 is removed from the holder 55, the detector 53a is provided in the second link 53. The detector 53a is, for example, an optical sensor, but is not limited thereto. The detector 53a may be a magnetic sensor, a mechanical switch, a current detection sensor, or a combination of these.

In this embodiment, the storage 351 stores the information on the pivot position PP. When operating the robot arm 50 based on an operation input to the joystick 62 after the instrument 2 is removed, the first control device 310 controls the operation of the robot arm 50 to maintain a distance between the pivot position PP and the holder 55. Specifically, the first control device 310 receives a command input through the joystick 62 from the surgical staff and controls the robot arm 50 such that the holder 55 moves with the pivot position PP as a center point and such that the distance between the holder 55 and the pivot position PP is maintained. In addition, the orientation of the instrument attachment surface of the holder 55 is controlled such that the shaft 2c of the instrument 2 passes through the pivot position PP when the instrument 2 is attached. The joystick 62 is urged by a spring or the like so as to return to a reference position when the surgical staff releases the joystick, and the robot arm 50 operates only when the surgical staff operates the joystick 62.

In this embodiment, when the arm operation unit 60 is operated, the display 220 of the cart 210 and the touch panel 130 of the remote control apparatus 200 display that the robot arm 50 is being operated. Specifically, while the robot arm 50 is in operation, the display 220 of the cart 210 and the touch panel 130 of the remote control apparatus 200 display that the robot arm 50 is being operated with the arm operation unit 60 and that the robot arm 50 is being moved to an instrument replacement position. The displayed portion may be displayed on the display 22a of the input 22 and the monitor 140, in addition to the display 220 and the touch panel 130. The display 220, the touch panel 130, the display 22a, and the monitor 140 are examples of a display.

FIG. 24 is a schematic view showing a state in which the replacement instrument 2A is attached to the holder 55. Since the distance between the holder 55 and the pivot position PP is maintained from the state of FIG. 22, and the holder 55 is maintained such that the shaft 2c passes through the pivot position PP when the instrument 2A is attached, the surgical staff can stop the robot arm 50 at any position, and locate the distal end of the instrument 2A inside the trocar T by simply attaching the instrument 2A to the holder 55.

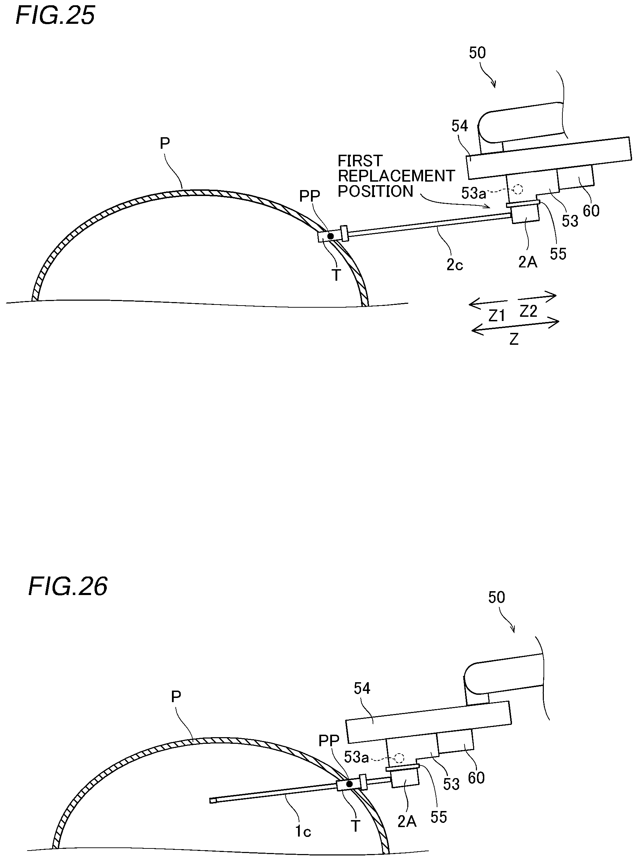

FIG. 25 is a schematic view showing a state in which the enable switch 61 of the arm operation unit 60 is operated to move the instrument 2A after replacement to the same inclination as the stored inclination of the instrument 2 before replacement. In this embodiment, when the enable switch 61 is operated with the instrument 2A attached to the holder 55 at the second replacement position, the first control device 310 controls the operation of the robot arm 50 to move the holder 55 from the second replacement position to the first replacement position. In addition, when the enable switch 61 is operated with the instrument 2A attached to the holder 55, the first control device 310 controls the operation of the robot arm 50 based on the pivot position PP and the distal end position of the instrument 2 at the time at which the instrument 2 has been removed. Specifically, when the surgical staff presses the enable switch 61, the first control device 310 performs a control to move the robot arm 50 toward the stored inclination of the instrument 2. At that time, the first control device 310 controls the operation of the robot arm 50 to maintain the distance between the holder 55 and the pivot position PP based on the enable switch 61 being operated. Therefore, the distal end of the instrument 2A can always operate while being located inside the trocar T. Since the distance between the pivot position PP and the holder 55 is controlled to be always constant, a position at which the instrument 2A has been moved to the same inclination as the inclination of the instrument 2 before replacement is the same as the first replacement position. In addition, since the shaft 2c of the instrument 2A always passes through the pivot position PP, no force is applied to the abdominal wall of the patient P through the shaft 2c even when the instrument 2A is left inserted in the trocar T.

The robot arm 50 operates only while the enable switch 61 is pressed. In this embodiment, the first control device 310 performs a control to disable an operation on the joystick 62 until the posture of the instrument 2A matches the stored posture of the instrument 2 with the instrument 2 attached. Specifically, the first control device 310 does not receive operations by the remote control apparatus 200, the joystick 62, or other buttons, switches, etc. of the arm operation unit 60 during the operation of matching the posture of the instrument 2A to the stored posture of the instrument 2. The first control device 310 controls the robot arm 50 to move from the posture when the instrument 2A is attached to the position at which the inclination of the shaft 2c of the instrument 2A becomes the stored inclination of the instrument 2 in the shortest distance. A path along which the instrument 2A moves to the stored inclination of the instrument 2 may be controlled such that the robot arm 50 moves in the shortest distance as described above, or the robot arm 50 may be operated such that the instrument 2A traces a path in reverse from a position at which the instrument 2 is removed to a position at which the instrument 2A is attached. In addition, intermediate points may be set in advance, and the instrument 2A may be operated to pass through the intermediate points.

In a mode in which the instrument 2A is moved to the stored inclination of the instrument 2 with the enable switch 61, pressing the mode switching button 64 enables the robot arm 50 to be operated with the joystick 62. The mode switching button 64 is used when the replacement of the instrument 2 with the instrument 2A is canceled without carrying out a guide tool change described below.

In this embodiment, the touch panel 130 of the remote control apparatus 200 receives an input of an offset amount at the time of movement from the second replacement position. When the enable switch 61 is operated with the instrument 2A attached to the holder 55 at the second replacement position, the first control device 310 controls the operation of the robot arm 50 to move the holder 55 from the second replacement position to a position offset from the first replacement position by the received offset amount along the longitudinal axis of the instrument 2 when the instrument 2 is removed. Specifically, by operating the touch panel 130 of the remote control apparatus 200, a position to which the distal end of the instrument 2A moves by operating the enable switch 61 can be offset in the Z direction. As an example, the offset amount can be set within a range of +10 mm to -10 mm in the Z direction from the stored distal end position of the instrument 2. The touch panel 130 is an example of a user input.

FIG. 26 is a schematic view showing a state in which the instrument 2A is inserted into a surgical area before replacement by a guide tool change function after the instrument 2A has been moved to the stored inclination of the instrument 2. When the instrument 2A has been moved to the stored inclination of the instrument 2, the guide tool change function can be operated. The guide tool change function refers to a function of moving the distal end of the instrument 2A after replacement to a predetermined distance before the distal end position of the instrument 2 before replacement by operating only the linear motion mechanism 54. The guide tool change function allows the surgical staff to easily and quickly move the instrument 2A after replacement to the surgical area before replacement.

The surgical staff simultaneously presses the enable switch 61 and the linear switch 63a such that only the linear motion mechanism 54 operates, and the distal end of the instrument 2A moves to the predetermined distance before the distal end position of the instrument 2 before replacement. The predetermined distance is, for example, about 3 mm, but is not limited to this. Each of the instruments 2 and 2A includes an internal memory that stores information regarding the instrument, such as the type of instrument, the number of uses of the instrument, and dimensional information such as the instrument length. The first control device 310 acquires length information of the instrument 2A from the memory, for example, and moves the instrument 2A to the predetermined distance before the distal end position of the instrument 2 before replacement based on the information. When the movement is completed, the first control device 310 deletes the stored information about the distal end position of the instrument 2 and the shaft 2c. After that, the doctor can operate the instrument 2A with the operation unit 110 according to the normal sequence.

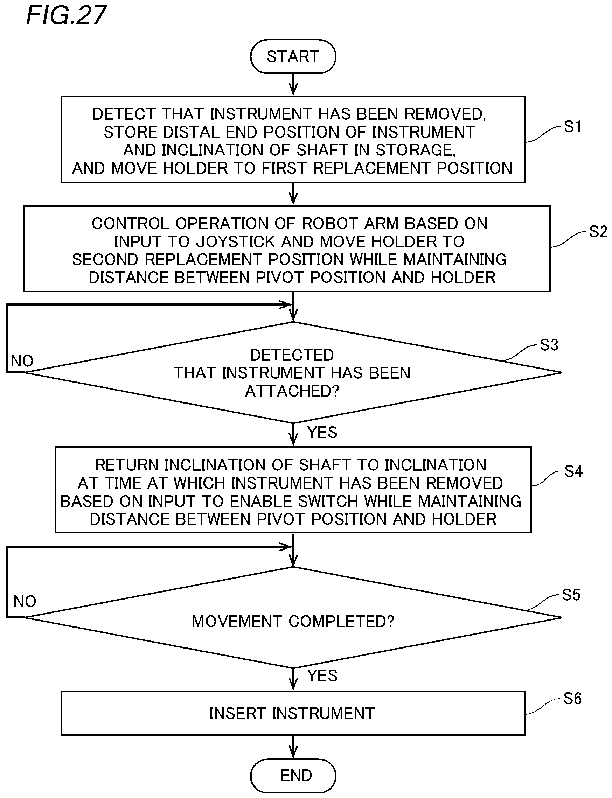

FIG. 27 is a control flow diagram according to this embodiment. In step S1, the first control device 310 detects that the instrument 2 has been removed, and stores the distal end position of the instrument 2 and the inclination of the shaft 2c in the storage 351. In addition, when the detector 53a detects that the instrument 2 has been removed, the first control device 310 moves the holder 55 to the first replacement position. Next, in step S2, the first control device 310 controls the operation of the robot arm 50 based on an input to the joystick 62 by the surgical staff, and moves the holder 55 from the first replacement position to the second replacement position, which is the replacement position of the instrument 2, while maintaining the distance between the pivot position PP and the holder 55. Then, in step S3, the first control device 310 determines whether or not it has been detected that the instrument 2A has been attached. When it has been detected that the instrument 2A has been attached, in step S4, the first control device 310 controls the robot arm 50 based on an input to the enable switch 61 by the surgical staff to return the inclination of the shaft 2c to the stored inclination at the time at which the instrument 2 has been removed. That is, the robot arm 50 is controlled to move the holder 55 from the second replacement position to the first replacement position. Then, in step S5, it is determined whether or not the movement is completed. Between step S2 and step S5, the linear motion mechanism 54 holds the second replacement position. Then, in step S6, the first control device 310 inserts the instrument 2A by the guide tool change.

### Advantages of This Embodiment

In the surgical robot 100, the first control device 310 is configured or programmed to perform a control to move the holder 55 to the first replacement position when the detector 53a detects that the instrument 2 has been removed, and move the holder 55 from the first replacement position to the second replacement position based on an input to the joystick 62. Accordingly, when the surgical staff removes the instrument 2 in order to replace the instrument 2 during surgery, the robot arm 50 can be operated using the joystick 62 arranged on the robot arm 50. Thus, the surgical staff can move the robot arm 50 to a posture in which the instrument 2 can be easily replaced. Consequently, the surgical staff can easily and quickly replace the instrument.

The arm operation unit 60 further includes the operator-operated enable switch 61. The first control device 310 is configured or programmed to control the operation of the robot arm 50 to move the holder 55 from the second replacement position to the first replacement position when the enable switch 61 is operated with the instrument 2A attached to the holder 55 at the second replacement position. Accordingly, the holder 55 can be easily moved from the second replacement position to the first replacement position simply by operating the enable switch 61.

The surgical robot 100 includes the storage 351 to store the information on the pivot position PP. The first control device 310 is configured or programmed to control the operation of the robot arm 50 based on the pivot position PP and the distal end position of the instrument 2 at the time at which the instrument 2 has been removed when the enable switch 61 is operated with the instrument 2A attached to the holder 55. Accordingly, the surgical staff can move the instrument 2A after replacement to the pivot position PP and the insertion posture of the instrument 2 at the time at which the instrument 2 before replacement has been removed. Consequently, the surgical staff can easily and quickly begin inserting the instrument 2A after replacement into the patient.

The first control device 310 is configured or programmed to control the operation of the robot arm 50 to maintain the distance between the pivot position PP and the holder 55 based on the enable switch 61 being operated. Accordingly, the surgical staff can operate the robot arm 50 while the distance of the instrument 2A in a direction of insertion into the body of the patient is maintained with the instrument 2A attached. Therefore, the instrument 2A can be moved to the insertion posture without adjusting the distance of the instrument 2A in the direction of insertion into the body of the patient.

The first control device 310 is configured or programmed to perform a control to disable an operation on the joystick 62 until the posture of the instrument 2A matches the stored posture of the instrument 2 with the instrument 2A attached. Accordingly, any erroneous operation on the joystick 62 is disabled, and thus the instrument 2A can be accurately moved to the insertion posture without readjustment.

The surgical robot 100 can move the instrument 2A to a position of insertion into the body of the patient only while the surgical staff is pressing the enable switch 61.

The surgical robot 100 is configured to control the operation of the robot arm 50 to maintain the distance between the pivot position PP and the holder 55 when the instrument 2 is removed and the robot arm 50 is operated based on an operation input to the joystick 62. Accordingly, the surgical staff can operate the robot arm 50 while the distance of the instrument 2A in the direction of insertion into the body of the patient is maintained until the instrument 2A is attached. Therefore, the instrument 2A can be attached without adjusting the distance of the instrument 2A in the direction of insertion into the body of the patient when the instrument 2A is attached. Consequently, the surgical staff can attach the instrument 2A more easily and more quickly.

The surgical robot 100 includes the linear motion mechanism 54 at the distal end of the robot arm 50, and the second link 53 attached so as to be linearly movable with respect to the linear motion mechanism 54, and the holder 55 and the arm operation unit 60 are each engaged with the second link 53. Accordingly, after the instrument 2 is replaced and the instrument 2A is moved to the insertion position, the surgical staff can perform the insertion operation of the holder 55 engaged with the second link 53 of the linear motion mechanism 54, the instrument 2A attached to the holder 55, and the arm operation unit 60 engaged with the holder 55 as a whole when operating the arm operation unit 60 such that the linear motion mechanism 54 inserts the instrument 2A into the body of the patient. Consequently, the surgical staff can easily and accurately insert the instrument 2A.

The first control device 310 is configured or programmed to cause the linear motion mechanism 54 to move the holder 55 to the position that is a certain distance away from the pivot position PP when the detector 53a detects that the instrument 2 has been removed. Accordingly, the holder 55 is automatically moved to the position that is a certain distance away from the pivot position PP, and thus the need for the operator to move the holder 55 is eliminated.

The surgical robot 100 includes the joystick 62 in the arm operation unit 60, and the robot arm 50 is operated with the joystick 62. Accordingly, the surgical staff can intuitively operate the robot arm 50 with the joystick 62. Consequently, the surgical staff can easily and quickly replace the instrument.

The robotic surgical system 500 further includes the display 220 and the touch panel 130 to display the status of the surgical robot 100. The display 220 and the touch panel 130 are configured to display the status of the surgical robot 100 such as that the robot arm 50 is being operated when the arm operation unit 60 is operated. Accordingly, the surgical staff can check the status of the robot arm 50 even from a position away from the robot arm 50. Thus, a surgical staff other than the surgical staff performing the instrument replacement work can share the status of the instrument replacement. In particular, when a plurality of displays are provided, the status of the instrument replacement can be more easily shared. Consequently, it contributes to improving the efficiency of the surgical procedure.

In the robotic surgical system 500, the touch panel 130 of the remote control apparatus 200 receives an input of the offset amount at the time of movement from the second replacement position. The first control device 310 is configured or programmed to, when the enable switch 61 is operated with the instrument 2A attached to the holder 55 at the second replacement position, control the operation of the robot arm 50 to move the holder 55 from the second replacement position to the position offset from the first replacement position by the received offset amount along the longitudinal axis of the instrument 2 when the instrument 2 is removed. Accordingly, during the movement, a difference in the movement position due to a difference in the length of the instrument can be finely adjusted simply by operating the enable switch 61.

### Modified Examples

The embodiment disclosed this time must be considered as illustrative in all points and not restrictive. The scope of the present disclosure is not shown by the above description of the embodiment but by the scope of claims for patent, and all modifications or modified examples within the meaning and scope equivalent to the scope of claims for patent are further included.

While the first control device 310, the storage 311, the second control device 350, and the storage 351 are arranged in the surgical robot 100 in the aforementioned embodiment, the present disclosure is not limited to this. In the present disclosure, all or at least one and less than all of the first control device 310, the storage 311, the second control device 350, and the storage 351 may alternatively be arranged outside the surgical robot 100.

While the information regarding the posture at the time at which the instrument 2 has been removed is stored in the storage 351 in the aforementioned embodiment, the present disclosure is not limited to this. For example, the information regarding the posture at the time at which the instrument 2 has been removed may alternatively be stored in the storage 311. Alternatively, it may be stored in both the storage 311 and the storage 351. Alternatively, the information regarding the posture at the time at which the instrument 2 has been removed may be stored in either the storage 311 or the storage 351, and may be transmitted from one to the other and stored therein as necessary for control purposes.

While the pivot position PP is stored in the storage 351 before the instrument 2 is replaced in the aforementioned embodiment, the present disclosure is not limited to this. The pivot position PP may alternatively be stored in both the storage 311 and the storage 351. Alternatively, the pivot position PP may be stored in either the storage 311 or the storage 351, the information regarding the posture at the time at which the instrument 2 has been removed may be transmitted from one to the other, and the pivot position PP may be stored therein as necessary for control purposes. Furthermore, the pivot position PP may alternatively be changed between the first instrument replacement and the second instrument replacement.

While the first control device 310 controls the operation of the robot arm 50 to maintain the distance between the pivot position PP and the holder 55 in the aforementioned embodiment, the present disclosure is not limited to this. The first control device 310 may alternatively control the robot arm 50 to operate at a distance greater than the initial distance between the pivot position PP and the holder 55. For example, the first control device 310 may control the distance to be varied within a range greater than the initial distance by up to 10 mm.

While the other operations are disabled when the enable switch 61 is being operated in the aforementioned embodiment, the present disclosure is not limited to this. In the present disclosure, a plurality of enable switches 61 may alternatively be arranged on the arm operation unit 60, and when the plurality of enable switches 61 are operated simultaneously, the other operations may alternatively be disabled. Alternatively, when any one of the plurality of enable switches 61 is operated, the other operations may be disabled.

While the enable switch 61 is operated to operate the robot arm 50 in the aforementioned embodiment, the present disclosure is not limited to this. In the present disclosure, another switch on the arm operation unit may alternatively have the same function, or a dedicated switch may alternatively be provided.

While the display 220 and the touch panel 130 display that the robot arm 50 is being operated in the aforementioned embodiment, the present disclosure is not limited to this. In the present disclosure, the display 22a and/or the monitor 140 may alternatively display that the robot arm 50 is being operated. In addition to displaying the message, a sound may be used to notify the operator that the robot arm 50 is being operated.

While the offset amount from the second replacement position is changed by inputting the offset amount at the time of movement from the second replacement position through the touch panel 130 in the aforementioned embodiment, the present disclosure is not limited to this. For example, the offset amount at the time of movement from the second replacement position may alternatively be fixed.

The functionality of the elements disclosed herein may be implemented using circuitry or processing circuitry that includes general purpose processors, special purpose processors, integrated circuits, application specific integrated circuits (ASICs), conventional circuitry and/or combinations thereof that are configured or programmed to perform the disclosed functionality. Processors are considered processing circuitry or circuitry as they include transistors and other circuitry therein. In the present disclosure, the circuitry, units, or means are hardware that carries out the recited functionality or hardware that is programmed to perform the recited functionality. The hardware may be hardware disclosed herein or other known hardware that is programmed or configured to carry out the recited functionality. When the hardware is a processor that may be considered a type of circuitry, the circuitry, means, or units are a combination of hardware and software, and the software is used to configure the hardware and/or processor.

### Aspects

It will be appreciated by those skilled in the art that the exemplary embodiments described above are specific examples of the following aspects.

### (Item 1)

A surgical robot comprising:
a robot arm including a holder to allow a first instrument to be attached thereto and detached therefrom;
an arm operation unit on the robot arm including an operation tool to operate the robot arm;
a detector in the holder to detect attachment and detachment of the first instrument to and from the holder; and
a controller configured or programmed to move the holder to a first replacement position when the detector detects that the first instrument has been removed; wherein
the controller is configured or programmed to perform a control to move the holder from the first replacement position to a second replacement position based on an input to the operation tool.

### (Item 2)

The surgical robot according to item 1, wherein
the arm operation unit further includes an operator-operated input element; and
the controller is configured or programmed to control operation of the robot arm to move the holder from the second replacement position to the first replacement position when the input element is operated with a second instrument attached to the holder at the second replacement position.

### (Item 3)

The surgical robot according to item 2, comprising:
a storage to store information on a pivot position; wherein
the controller is configured or programmed to control the operation of the robot arm based on the pivot position and a distal end position of the first instrument at a time at which the first instrument has been removed when the input element is operated with the second instrument attached to the holder.

### (Item 4)

The surgical robot according to item 3, wherein the controller is configured or programmed to control the operation of the robot arm to maintain a distance between the pivot position and the holder based on the input element being operated.

### (Item 5)

The surgical robot according to any one of items 2 to 4, wherein the controller is configured or programmed to perform a control to disable an operation on the operation tool until a posture of the second instrument matches a stored posture of the first instrument with the second instrument attached.

### (Item 6)

The surgical robot according to any one of items 2 to 5, wherein the input element includes an enable switch on the arm operation unit.

### (Item 7)

The surgical robot according to any one of items 1 to 6, comprising:
a storage to store information on a pivot position; wherein
the controller is configured or programmed to control operation of the robot arm to maintain a distance between the pivot position and the holder when the first instrument is removed and the robot arm is operated based on the operation input to the operation tool.

### (Item 8)

The surgical robot according to any one of items 1 to 7, wherein
the robot arm includes:
   a linear motion mechanism at a distal end of the robot arm; and
   a movable element attached so as to be linearly movable with respect to the linear motion mechanism; and
the holder and the arm operation unit are each engaged with the movable element.

### (Item 9)

The surgical robot according to item 8, wherein the controller is configured or programmed to move the linear motion mechanism to a position at which the holder is a certain distance away from a pivot position when the detector detects that the first instrument has been removed.

### (Item 10)

The surgical robot according to any one of items 1 to 9, wherein
the operation tool includes a joystick; and
the controller is configured or programmed to control the robot arm based on an operation input to the joystick.

### (Item 11)

A robotic surgical system comprising the surgical robot according to any one of items 1 to 10, and an operation apparatus to operate the surgical robot.

### (Item 12)

The robotic surgical system according to item 11, comprising:
one or more displays to display a status of the surgical robot; wherein
the one or more displays are configured to display that the robot arm is being operated when the arm operation unit is operated.

### (Item 13)

The robotic surgical system according to item 12, wherein
the operation apparatus includes a user input to receive an input of an offset amount at a time of movement from the second replacement position;
the arm operation unit further includes an operator-operated input element; and
the controller is configured or programmed to, when the input element is operated with a second instrument attached to the holder at the second replacement position, control operation of the robot arm to move the holder from the second replacement position to a position offset from the first replacement position by a received offset amount along a longitudinal axis of the first instrument when the first instrument is removed.

### (Item 14)

A control method for a surgical robot in which an instrument is replaced during surgery, the control method comprising:
performing a control to detect that the instrument has been removed during the surgery;
performing a control to move, to a first replacement position, a holder configured to allow the instrument to be attached and detached thereto and therefrom when it is detected that the instrument has been removed; and
performing a control to move the holder from the first replacement position to a second replacement position based on an input to an operation tool configured to operate a robot arm of the surgical robot.

### (Item 15)

A program for the control method for the surgical robot according to item 14.

### (Item 16)

A storage medium configured to store the program for the control method for the surgical robot according to item 15.

## Claims

1. A surgical robot (100) comprising:
a robot arm (50) including a holder (55) to allow a first instrument (2) to be attached thereto and detached therefrom;
an arm operation unit (60) on the robot arm including an operation tool (62) to operate the robot arm;
a detector (53a) in the holder to detect attachment and detachment of the first instrument to and from the holder; and
a controller (310) configured or programmed to move the holder to a first replacement position when the detector detects that the first instrument has been removed; wherein
the controller is configured or programmed to perform a control to move the holder from the first replacement position to a second replacement position based on an input to the operation tool.

2. The surgical robot according to claim 1, wherein
the arm operation unit further includes an operator-operated input element (61); and
the controller is configured or programmed to control operation of the robot arm to move the holder from the second replacement position to the first replacement position when the input element is operated with a second instrument (2A) attached to the holder at the second replacement position.

3. The surgical robot according to claim 2, comprising:
a storage (351) to store information on a pivot position (PP); wherein
the controller is configured or programmed to control the operation of the robot arm based on the pivot position and a distal end position of the first instrument at a time at which the first instrument has been removed when the input element is operated with the second instrument attached to the holder.

4. The surgical robot according to claim 3, wherein the controller is configured or programmed to control the operation of the robot arm to maintain a distance between the pivot position and the holder based on the input element being operated.

5. The surgical robot according to any one of claims 2 to 4, wherein the controller is configured or programmed to perform a control to disable an operation on the operation tool until a posture of the second instrument matches a stored posture of the first instrument with the second instrument attached.

6. The surgical robot according to any one of claims 2 to 5, wherein the input element includes an enable switch (61) on the arm operation unit.

7. The surgical robot according to any one of claims 1 to 6, comprising:
a storage (351) to store information on a pivot position (PP); wherein
the controller is configured or programmed to control operation of the robot arm to maintain a distance between the pivot position and the holder when the first instrument is removed and the robot arm is operated based on the operation input to the operation tool.

8. The surgical robot according to any one of claims 1 to 7, wherein
the robot arm includes:
a linear motion mechanism (54) at a distal end of the robot arm; and
a movable element (53) attached so as to be linearly movable with respect to the linear motion mechanism; and
the holder and the arm operation unit are each engaged with the movable element.

9. The surgical robot according to claim 8, wherein the controller is configured or programmed to cause the linear motion mechanism to move the holder to a position that is a certain distance away from a pivot position when the detector detects that the first instrument has been removed.

10. The surgical robot according to any one of claims 1 to 9, wherein
the operation tool includes a joystick (62); and
the controller is configured or programmed to control the robot arm based on an operation input to the joystick.

11. A robotic surgical system (500) comprising the surgical robot according to any one of claims 1 to 10, and an operation apparatus to operate the surgical robot.

12. The robotic surgical system according to claim 11, comprising:
one or more displays (22a, 130, 140) to display a status of the surgical robot; wherein
the one or more displays are configured to display that the robot arm is being operated when the arm operation unit is operated.

13. The robotic surgical system according to claim 12, wherein
the operation apparatus includes a user input (130) to receive an input of an offset amount at a time of movement from the second replacement position;
the arm operation unit further includes an operator-operated input element (61); and
the controller is configured or programmed to, when the input element is operated with a second instrument (2A) attached to the holder at the second replacement position, control operation of the robot arm to move the holder from the second replacement position to a position offset from the first replacement position by a received offset amount along a longitudinal axis of the first instrument when the first instrument is removed.

14. A control method for a surgical robot (100) in which an instrument (2) is replaced during surgery, the control method comprising:
performing a control to detect that the instrument has been removed during the surgery;
performing a control to move, to a first replacement position, a holder (55) configured to allow the instrument to be attached and detached thereto and therefrom when it is detected that the instrument has been removed; and
performing a control to move the holder from the first replacement position to a second replacement position based on an input to an operation tool (62) configured to operate a robot arm (50) of the surgical robot.

15. A program for the control method for the surgical robot (100) according to claim 14.

16. A storage medium configured to store the program for the control method for the surgical robot (100) according to claim 15.
